(19) Europäisches Patentamt
European Patent Office
Office européen des brevets

(11) Veröffentlichungsnummer: **0 048 411**
**B1**

(12) # EUROPÄISCHE PATENTSCHRIFT

(45) Veröffentlichungstag der Patentschrift: **12.11.86**

(21) Anmeldenummer: **81107180.2**

(22) Anmeldetag: **11.09.81**

(51) Int. Cl.⁴: **C 07 C 119/10,**
C 07 C 43/313, C 07 C 41/50,
C 07.C 59/88, C 07 C 121/76

(54) **Verfahren zur Herstellung von 3-Brom-4-fluor-benzaldehydacetalen, neue Zwischenprodukte hierfür und Verfahren zu deren Herstellung.**

(30) Priorität: **24.09.80 DE 3035921**

(43) Veröffentlichungstag der Anmeldung:
**31.03.82 Patentblatt 82/13**

(45) Bekanntmachung des Hinweises auf die Patenterteilung:
**12.11.86 Patentblatt 86/46**

(84) Benannte Vertragsstaaten:
**BE CH DE FR GB IT LI NL**

(56) Entgegenhaltungen:
**EP-A-0 024 624**
**FR-A-2 405 241**

(73) Patentinhaber: **BAYER AG**
**Konzernverwaltung RP Patentabteilung**
**D-5090 Leverkusen 1 Bayerwerk (DE)**

(72) Erfinder: **Maurer, Fritz, Dr.**
**Roeberstrasse 8**
**D-5600 Wuppertal 1 (DE)**
Erfinder: **Priesnitz, Uwe, Dr.**
**Severinstrasse 58**
**D-5650 Solingen (DE)**
Erfinder: **Riebel, Hans-Jochem, Dr.**
**In der Beek 92**
**D-5600 Wuppertal (DE)**
Erfinder: **Gallenkamp, Bernd, Dr.**
**Claudiusweg 5**
**D-5600 Wuppertal (DE)**
Erfinder: **Klauke, Erich, Dr.**
**Eichendorffweg 8**
**D-5063 Odenthal (DE)**

EP 0 048 411 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft ein neues Verfahren zur Herstellung von 3-Brom-4-fluor-benzaldehyd-acetalen und 3-Brom-4-fluor-benzaldimine als neue Zwischenprodukte hierfür und Verfahren zu deren Herstellung.

Es ist bekannt, daß man 4-Fluor-3-phenoxy-benzaldehyd, ein Zwischenprodukt für pestizid wirksame Pyrethroide, erhält, wenn man 4-Fluor-3-phenoxy-benzylbromid mit Hexamethylentetramin umsetzt und das Produkt dieser Umsetzung mit Säuren erhitzt (vgl. DE—OS 2 709 264). Die Ausbeute ist jedoch bei dieser Synthesemethode, wie auch bei der Herstellung der Ausgangsverbindung aus 4-Fluor-3-phenoxy-toluol und N-Brom-succinimid, unbefriedigend.

Wieter sind 3-Brom-4-fluor-benzaldehyd und Acetale hiervon sowie 4-Fluor-3-phenoxy-benzaldehyd-acetale als neue Zwischenprodukte zur Herstellung von 4-Fluor-3-phenoxy-benzaldehyd Gegenstand einer eigenen älteren, nicht vorveröffentlichten deutschen Patentanmeldung (vgl. P 29 33 979).

(1) Es wurde ein Verfahren zur Herstellung von 3-Brom-4-fluor-benzaldehyd-acetalen der Formel I gefunden,

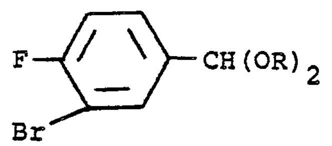

(1)

in welcher die Reste

R einzeln für $C_1$—$C_4$-Alkyl oder zusammen für $C_2$—$C_5$-Alkandiyl stehen, das dadurch gekennzeichnet ist, daß man

(a) 3-Brom-4-fluor-benzaldimine der Formel II

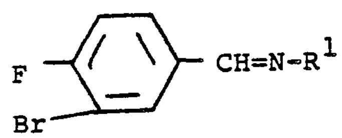

(II)

in welcher

$R^1$ für gegebenenfalls verzweigtes und/oder gegebenenfalls cyclisches und/oder gegebenenfalls substituiertes Alkyl oder für gegebenenfalls substituiertes Aryl steht, mit Alkoholen bzw. Alkandiolen der Formel III

$$(HO)_n—R \qquad (III)$$

in welcher

R die oben angegebene Bedeutung hat und

n für 1 oder 2 steht, in Gegenwart von Schwefelsäure bei Temperaturen zwischen 0 und 100°C umsetzt, oder

(b) 3-Brom-4-fluor-phenylglyoxylsäure der Formel IV

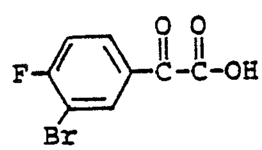

(IV)

mit Aminen der Formel, V

$$H_2N—R^1 \qquad (V)$$

in welcher

$R^1$ die oben angegebene Bedeutung hat, gegebenenfalls in Gegenwart eines Verdünnungsmittels bei Temperaturen zwischen 0 und 200°C umsetzt und die hierbei erhaltenen Rohprodukte, welche die Verbindungen der Formel (II) enthalten, mit Alkoholen der Formel (III) in Gegenwart katalytischer Mengen Schwefelsäure bei Temperaturen zwischen 0 und 100°C umsetzt;

(2) ferner wurden die 3-Brom-4-fluor-benzaldimine der Formel II gefunden,

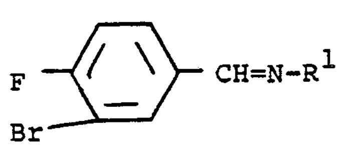

(II)

in welcher

$R^1$ für gegebenenfalls verzweigtes, gegebenenfalls cyclisches und gegebenenfalls substituiertes Alkyl oder für gegebenenfalls substituiertes Aryl steht;

(3) ferner wurde ein Verfahren zur Herstellung der neuen Verbindung der Formel (II) gefunden,

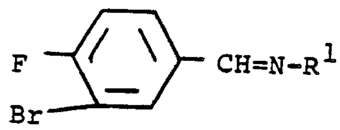

(II)

das dadurch gekennzeichnet ist, daß man

(a) 3-Brom-4-fluor-benzaldehyd der Formel VI

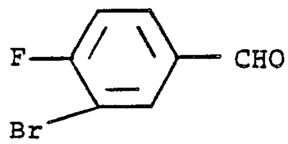

(VI)

mit Aminen der Formel (V) (oben) gegebenenfalls in Gegenwart eines Verdünngungmittels umsetzt, oder

(b) 3-Brom-4-fluor-phenylglyoxylsäure der Formel IV (oben) mit Aminen der Formel V (oben) gegebenenfalls in Gegenwart eines Verdünnungsmittels umsetzt.

Die 3-Brom-4-fluor-phenylglyoxylsäure der Formel (IV)

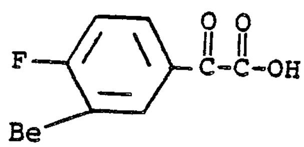

(IV)

wird hergestellt, indem man 3-Brom-4-fluor-benzoylcyanid der Formel (VII)

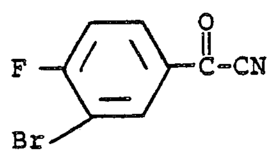

(VII)

mit Salzsäure der Schwefelsäure umsetzt;

Das vorgenannte 3-Brom-4-fluor-benzoylcyanid der Formel (VII)

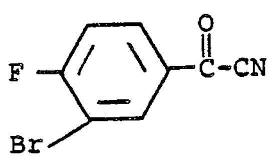

(VII)

wird dadurch erhalten, indem man 3-Brom-4-fluor-benzoylhalogenide der Formel (VIII)

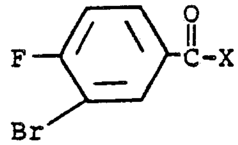

(VIII)

in welcher

X für Fluor, Chlor oder Brom steht, mit Natriumcyanid oder Kaliumcyanid umsetzt.

Überraschenderweise können 3-Brom-4-fluor-benzaldehyd-acetale nach dem erfindungsgemäßen Verfahren über die neuen 3-Brom-4-fluor-benzaldimine auf einfachere Weise und in höheren Ausbeuten als nach den oben erwähnten Methoden hergestellt werden. Vorteile des neuen Verfahrens liegen vor allem darin, daß billige Ausgangsstoffe bzw. Reaktionskomponenten verwendet werden können, keine Reduktions- bzw. Oxidationsmittel erforderlich sind und die Acetalisierung ohne zusätzliche Hilfsstoffe wie Orthoester möglich ist.

Das unter (1) dargelegte neue Verfahren ("Verfahren (1)") kann bei Verwendung von N-Phenyl-3-brom-4-fluor-benzaldimin und Ethanol (a) bzw. von 3-Brom-4-fluor-phenylglyoxylsäure und Anilin sowie anschließend Ethanol (b) durch folgendes Reaktionsschema skizziert werden:

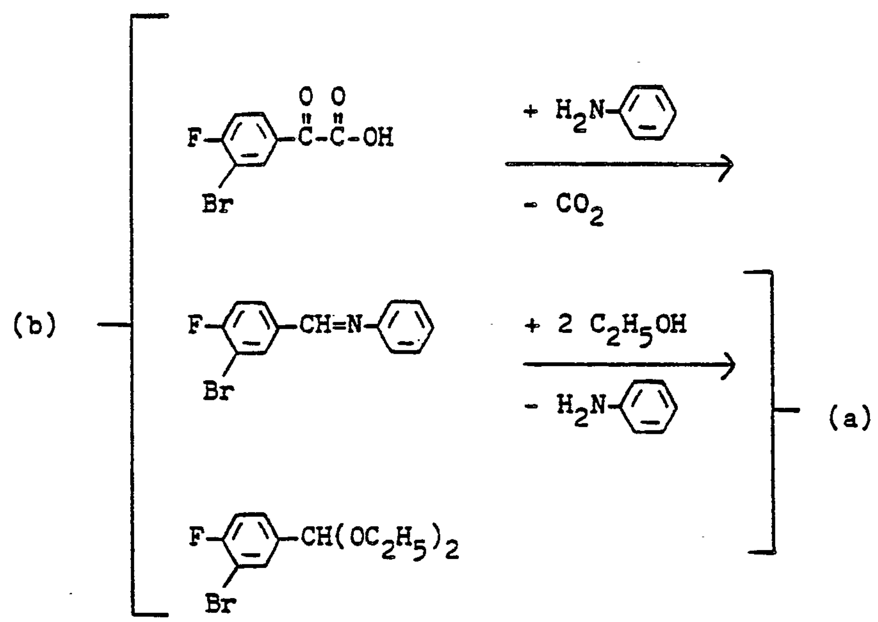


Die oben unter (a) dargelegte Variante von Verfahren (1) wird bei Temperaturen zwischen 0 und 100°C, vorzugsweise zwischen 10 und 50°C durchgeführt.

Die Alkohole bzw. Alkandiole, welche bei dieser Verfahrensvariante als Ausgangsstoffe verwendet werden, sind durch Formel (III) definiert.

Als Beispiele seien genannt:

Methanol, Ethanol, n- und iso-Propanol, n-, iso-, sec- und tert-Butanol sowie Ethan-1,2-diol, Propan-1,3-diol und 2,2-Dimethylpropan-1,3-diol.

Methanol, Ethanol und Ethan-1,2-diol werden als Ausgangsstoffe bevorzugt.

Die Alkohole der Formel (III) werden zur Verdünnung der Reaktionsgemische im Überschuß eingesetzt. Im allgemeinen setzt man je Mol Aldimin der Formel (II) bis zu 80 Mol, vorzugsweise bis zu 40 Mol Alkohol der Formel (III) ein.

Je Mol Aldimin der Formel (II) werden weiter zwischen 0,5 und 1,5 Mol, vorzugsweise zwischen 0,5 und 1 Mol Schwefelsäure verwendet.

In einer bevorzugten Ausführungsform der Verfahrensvariante (a) wird das 3-Brom-4-fluor-benzaldimin der Formel (II) in einem Alkohol der Formel (III) vorgelegt und zu dieser Lösung wird die Schwefelsäure langsam eindosiert. Das Reaktionsgemisch wird bis zum Ende der Umsetzung gerührt.

Die Aufarbeitung kann nach üblichen Methoden durchgeführt werden. Beispielsweise wird der Alkohol unter vermindertem Druck abdestilliert, der Rückstand mit einem mit Wasser praktisch nicht mischbaren organischen Lösungsmittel, wie z.B. Toluol, verdünnt und vom Ungelösten abgesaugt. Das Filtrat wird gewaschen (z.B. mit wässriger Kaliumcarbonatlösung), getrocknet, filtriert und eingedampft.

Man erhält dann die Produkte der Formel (I) durch Vakuumdestillation des Rückstandes.

Die erste Stufe der unter (b) dargelegten Variante von Verfahren (1) wird vorzugsweise unter Verwendung von Verdünnungsmitteln durchgeführt. Als Verdünnungsmittel kommen praktisch alle inerten organischen Lösungsmittel in Frage.

Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glycoldimethylether und Diglycoldimethylether, Tetrahydrofuran und Dioxan, sowie Amide, wie z.B. Dimethylformamid und Dimethylacetamid.

Die Reaktionstemperatur kann innerhalb eines größeren Bereichs variiert werden. Im allgemeinen arbeitet man zwischen 0 und 200°C, vorzugsweise bei 50 bis 150°C.

Je Mol Phenylglyoxylsäure der Formel (IV) werden im allgemeinen 0,9 bis 1,5 Mol, vorzugsweise 0,95 bis 1,2 Mol Amin der Formel (V) eingesetzt.

In einer bevorzugten Ausführungsform der ersten Stufe von Verfahrensvariante (b) wird 3-Brom-4-fluor-phenylglyoxylsäure (IV) in einem der oben angegebenen Verdünnungsmittel bei Temperaturen zwischen 50 und 100°C vorgelegt und das Amin der Formel (V) wird bei dieser Temperatur dazu gegeben. Das Reaktionsgemisch wird bis zum Ende der Decarboxylierung erhitzt und dann durch Destillation unter

vermindertem Druck das Lösungsmittel sorgfältig entfernt. Das hierbei als Rückstand erhaltene Rohprodukt der Formel (II) wird dann wie für Variante (a) beschrieben in das Produkt der Formel (I) umgewandelt.

Die bei Verfahren (1) als Ausgangsstoffe zu verwendenden 3-Brom-4-fluor-benzaldimine sind durch Formel (II), die entsprechenden Amine durch Formel (V) definiert. Vorzugsweise steht in diesen Formeln

$R^1$ für $C_5$—$C_6$-Cycloalkyl oder für gegebenenfalls durch Methyl, Ethyl, Chlor und/oder Nitro substituiertes Phenyl.

Als Beispiele für die Aldimine der Formel (II) seien genannt:

N-Cyclopentyl-, N-Cyclohexyl-, N-Phenyl-, N-(4-Methyl-phenyl)-, N-(3-Methyl-phenyl)-, N-(2-Methyl-phenyl)-, N-(4-Chlor-phenyl)-, N-(4-Nitro-phenyl)-, N-(3-Nitro-phenyl)- und N-(2,6-Diethyl-phenyl)-3-brom-4-fluor-benzaldimin.

Als Beispiele für die Amine der Formel (V) seien genannt:

Cyclopentylamin, Cyclohexylamin, Anilin, 2-Methyl-, 3-Methyl-, 4-Methyl-, 4-Chlor-, 4-Nitro-, 3-Nitro- und 2,6-Diethyl-anilin.

Das oben unter (3) dargelegte Verfahren zur Herstellung der neuen 3-Brom-4-fluor-benzaldimine ("Verfahren (3)") kann bei Verwendung von 3-Brom-4-fluor-benzaldehyd und Anilin (a) bzw. von 3-Brom-4-fluor-phenylglyoxylsäure und Anilin (b) als Ausgangsstoffen durch folgendes Reaktionsschema skizziert werden:

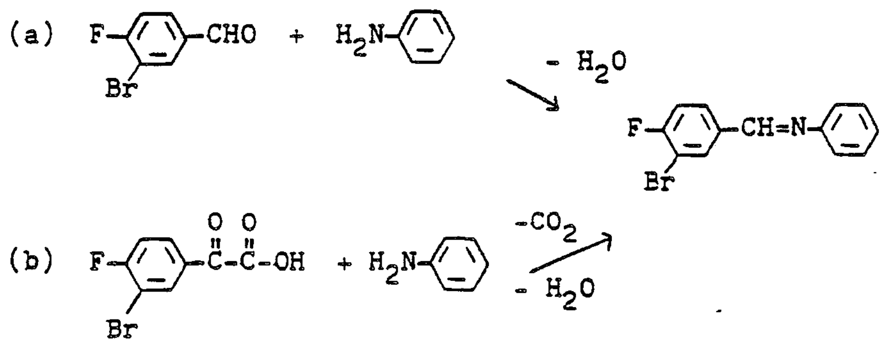


Als Verdünnungsmittel werden in beiden Varianten von Verfahren (3) vorzugsweise die oben für Verfahren (1) angegebenen Lösungsmittel verwendet.

Die Reaktionstemperatur kann bei beiden Varianten innerhalb eines größeren Bereichs variiert werden. Im allgemeinen arbeitet man zwischen 0 und 200°C, vorzugsweise bei 50 bis 150°C.

Je Mol 3-Brom-4-fluor-benzaldehyd bzw. 3-Brom-4-fluor-phenylglyoxylsäure werden im allgemeinen zwischen 0,9 und 1,5 Mol, vorzugsweise 0,95 bis 1,2-Mol Amin der Formel (V) eingesetzt.

In einer bevorzugten Ausführungsform der Variante (a) von Verfahren (3) wird 3-Brom-4-fluor-benzaldehyd mit einem Amin der Formel (V) in einem der oben angegebenen Verdünnungsmittel, vorzugsweise in einem zur azeotropen Wasserabscheidung geeigneten Lösungsmittel, wie z.B. Toluol, bis zum Ende der Umsetzung zum Sieden erhitzt, wobei vorzugsweise das als Koppelprodukt gebildete Wasser über einen Wasserabschieder entfernt wird. Die Produkte der Formel (II) werden durch sorgfältiges Abdestillieren der flüchtigen Komponenten unter vermindertem Druck isoliert.

Die Durchführung der Variante (b) von Verfahren (3) ist oben für die erste Stufe der Variante (b) von Verfahren (1) beschrieben.

Der als Ausgangsstoff zu verwendende 3-Brom-4-fluor-benzaldehyd (VI) ist Gegenstand einer vorgängigen Patentanmeldung (vgl. deutsche Patentanmeldung P 29 33 979).

Die alternativ als Ausgangsverbindung zu verwendende 3-Brom-4-fluor-phenylglyoxylsäure erhält man durch Umsetzung von 3-Brom-4-fluor-benzoylcyanid mit Salzsäure der Schwefelsäure, vorzugsweise Salzsäure. Die Temperatur wird hierbei im allgemeinen zwischen 0 und 100°C, vorzugsweise zwischen 20 und 80°C, gehalten. Je Mol 3-Brom-4-fluor-benzoylcyanid setzt man 1 bis 2 Liter einer der genannten Säuren in einer Konzentration zwischen 30 und 60 Gewichtsprozent ein. Die Säurekonzentration wird während der Umsetzung gegebenenfalls durch Einleiten von Chlorwasserstoffgas erhöht.

Zur Herstellung von 3-Brom-4-fluor-phenylglyoxylsäure (IV) wird die Mischung der Reaktionskomponenten bei 20 bis 30°C mehrere Stunden gerührt und dann mehrere Stunden bei 70 bis 80°C erhitzt. Beim Abkühlen des Reaktionsgemisches auf 20°C fällt 3-Brom-4-fluor-phenylglyoxylsäure in kristalliner Form aus und kann durch Absaugen isoliert werden.

Das zur obigen Herstellung von 3-Brom-4-fluor-phenylglyoxylsäure (IV) als Ausgangsprodukt benötigte 3-Brom-4-fluor-benzoylcyanid erhält man durch Umsetzung von 3-Brom-4-fluor-benzoylhalogeniden der Formel VIII (oben) mit Natriumcyanid oder Kaliumcyanid.

Diese Umsetzung wird bei Temperaturen zwischen 0 und 150°C, vorzugsweise zwischen 20 und 120°C durchgeführt.

Je Mol Halogenid der Formel (VIII) setzt man 0,9 bis 1,5 Mol, vorzugsweise 1 bis 1,2 Mol Alkalicyanid ein.

Zur Herstellung von 3-Brom-4-fluor-benzoylcyanid (VII) aus 3-Brom-4-fluor-benzoylhalogeniden (VIII) und NaCN oder KCN werden vorzugsweise die Reaktionskomponenten bei 20°C vermischt und das Gemisch wird bis zum Ende der Umsetzung auf Temperaturen zwischen 50 und 120°C erhitzt. Nach Abkühlen des Gemisches wird abgesaugt. Das im Filtrat befindliche Produkt der Formel (VII) erhält man in reiner Form durch Vakuumdestillation.

Man erhält beispielsweise ein Gemisch aus 3-Brom-4-fluorbenzoesäure-fluorid und -bromid, wenn man 4-Chlor-benzoylchlorid durch Umsetzung mit Kaliumfluorid in 4-Fluorbenzoylfluorid umwandelt und letzteres anschließend bromiert.

4-Chlor-benzoylchlorid wird mit Kaliumfluorid beispielsweise in Tetramethylensulfon bei Temperaturen zwischen 200 und 220°C umgesetzt und das Reaktionsgemisch destillativ aufgearbeitet. Man erhält 4-Fluor-benzoylfluorid vom Siedepunkt 53°C/20 mBar (Brechungsindex $n_D^{20}$:1,4792).

4-Fluor-benzoylfluorid wird mit elementarem Brom in Gegenwart von 1% Eisen (III) chlorid bei 70 bis 75°C umgesetzt. Bei einem Ansatz von 1 Mol erhält man nach Destillation 40 g unverändertes Ausgangsmaterial zurück und 182 g eines Gemisches aus 3-Brom-4-fluor-benzoylfluorid (Siedepunkt 82—83°C/15 mBar; Brechungsindex $n_D^{20}$:1,5315; Schmelzpunkt 32—34°C) und 3-Brom-4-fluorbenzoylbromid (Siedepunkt: 123°C/15 mBar; Schmelzpunkt 35—37°C).

Die 3-Brom-4-fluor-benzaldehyd-acetale der Formel I (oben) können zur Herstellung von 3-Phenoxy-4-fluor-benzaldehyd, welcher als Zwischenprodukt für Insektizide bekannt ist (vgl. DE—OS 2 709 264), verwendet werden, wobei 3-Phenoxy-4-fluor-benzaldehyd-acetale als Zwischenprodukte isoliert werden können.

Die Herstellung von 3-Phenoxy-4-fluor-benzaldehyd kann beispielsweise bei Verwendung von 3-Brom-4-fluor-benzaldehyd-diethylacetal und Kaliumphenolat als Ausgangsstoffen in einer ersten Stufe und bei einer als zweite Stufe durchzuführenden Acetalspaltung mit einer Säure, wie z.B. Salzsäure, durch folgendes Formelschema skizziert werden:

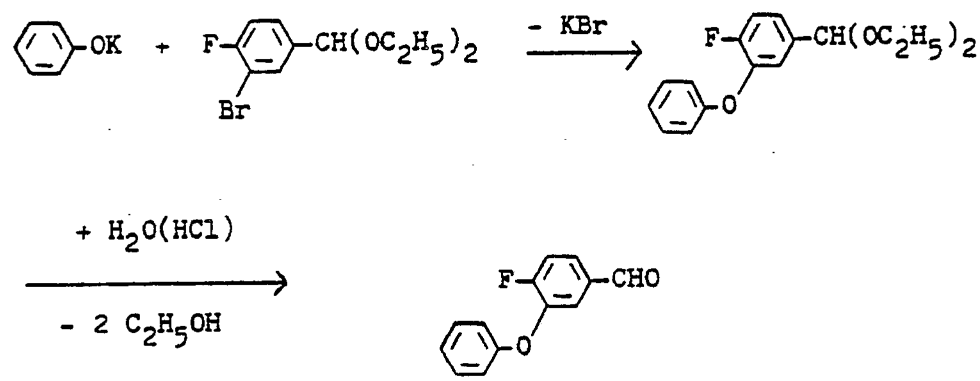


Alkali- bzw. Erdalkaliverbindungen von Phenolen, welche hierbei als Ausgangsstoffe verwendet werden können, sind z.B. Natrium-, Kalium- und Magnesium-phenolat.

Als Katalysatoren werden Kupfer oder Kupferverbindungen verwendet. Als Beispiele hierfür seien Kupfer, Kupfer(I)oxid, Kupfer(II)oxid, Kupfer(I)chlorid und Kupfer(I)bromid genannt.

Als Verdünnungsmittel werden vorzugsweise aprotisch polare Solventien verwendet. Beispiele hierfür sind Dimethylformamid, Dimethylacetamid, N-Methylpyrrolidon, Dimethylsulfoxid, Tetramethylensulfon, Hexamethylphosphorsäuretriamid und Bis-(2-methoxyethyl)-ether (Diglyme). Letzteres wird besonders bevorzugt.

Die Reaktionstemperatur wird zwischen 100 und 200°, vorzugsweise zwischen 130 und 170°C gehalten. Das Verfahren wird gewöhnlich unter Normaldruck durchgeführt.

Auf 1 Mol 3-Brom-4-fluor-benzaldehyd-acetal der Formel (I) setzt man 1 bis 2 Mol, vorzugsweise 1,2 bis 1,8 Mol Phenolat, 0,01 bis 0,5 Mol, vorzugsweise 0,1 bis 0,5 Mol Kupferkatalysator und 100 bis 500 ml Verdünnungsmittel ein.

In einer bevorzugten Ausführungsform der dargelegten Umsetzung wird das Phenolat in einem der oben angegebenen Verdünnungsmittel zusammen mit dem Kupferkatalysator vorgelegt und das Gemisch wird auf die Reaktionstemperatur aufgeheizt. Dann wird das 3-Brom-4-fluor-benzaldehydacetal zugetropft und das Reaktionsgemisch einige Stunden nachgerührt. Die Aufarbeitung kann nach üblichen Methoden erfolgen, beispielsweise durch Abdestillieren des Verdünnungsmittels unter vermindertem Druck, Lösen des Rückstandes in Toluol, Filtrieren, Waschen des Filtrats mit verdünnter Natronlauge und Destillieren unter vermindertem Druck. Man erhält so die 3-Phenoxy-4-fluor-benzaldehyd-acetale als farblose Flüssigkeiten.

Die Verseifung der Acetale zu 3-Phenoxy-4-fluor-benzaldehyd kann nach üblichen Methoden durchgeführt werden. In einer bevorzugten Verfahrensweise werden die Acetale mit einer verdünnten Mineralsäure, wie z.B. Salzsäure oder Schwefelsäure, vermischt und mehrere Stunden bei Temperaturen zwischen 20 und 60°C gerührt. Zur Aufarbeitung wird mit einem mit Wasser praktisch nicht mischbaren organischen Lösungsmittel, wie z.B. Toluol, extrahiert, die Extrakte werden mit Natriumhydrogencarbonatlösung und Wasser gewaschen und durch Destillation unter vermindertem Druck vom Lösungsmittel befreit. 3-Phenoxy-4-fluor-benzaldehyd verbleibt hierbei als öliger Rückstand.

Beispiel 1

4-Fluor-3-brom-benzoylcyanid:

177 g (3,6 Mol) Natriumcyanid und 663 g (3 Mol) 3-Brom-4-fluorbenzoylfluorid werden gemeinsam vorgelegt und aufgeheizt. Dabei kommt es bei ca. 80°C zu einer exothermen Reaktion, die durch leichtes Kühlen etwas abgefangen werden muß. Man läßt die Temperatur dabei bis auf 100°C ansteigen und rührt 1 Stunde bei 100°C. Die exotherme Reaktion hält sich ca. 10 Minuten, danach muß zusätzlich geheizt werden. Anschließend wird der Ansatz abgekühlt, die Salze abgesaugt und in gesättigter Eisensulfat-Lösung vernichtet und das Filtrat an einer Kolonne destilliert. Man erhält so 507 g (74% der Theorie) eines farblosen Öles mit dem Siedepunkt 126°C/20 Torr.

Beispiel 2

4-Fluor-3-brom-phenylglyoxylsäure:

a) Man läßt eine Mischung aus 228 g 4-Fluor-3-Brom-benzoylcyanid und 1625 ml konz. Salzsäure 20 Stunden bei Raumtemperatur rühren. Danach heizt man langsam auf 70—80°C und rührt 8 Stunden bei dieser Temperatur. Zur Auskristallisation wird das Reaktionsgemisch noch 17 Stunden bei Raumtemperatur gerührt, danach abgesaugt, mit 1000 ml Wasser nachgewaschen und im Vakuum bei 35—40°C 24 Stunden getrocknet. Man erhält so 246 g (99,6% der Theorie) 4-Fluor-3-brom-phenylglyoxylsäure als farbloses Pulver mit dem Schmelzpunkt 68—73°C.

b) In eine Lösung von 45,6 g (0,2 Mol) 4-Fluor-3-brom-benzoylcyanid in 200 ml konz. Salzsäure leitet man 5 Stunden einen schwachen Chlorwasserstoffstrom ein. Dabei steigt die Temperatur auf +30°C. Man rührt anschließend 6 Stunden bei 80°C, kühlt dann ab und saugt die ausgefallenen Kristalle ab. Man erhält 43 g (87% der Theorie) 4-Fluor-3-brom-phenyl-glyoxylsäure in Form farbloser Kristalle.

Beispiel 3a

4-Fluor-3-brom-benzaldehydanil

Zu einer Mischung aus 1300 ml Toluol oder Chlorbenzol und 247 g 4-Fluor-3-bromphenylglyoxylsäure tropft man bei 70—80°C 102,3 g (1,1 Mol) frisch destilliertes Anilin. Anschließend erhitzt man die Reaktionsmischung auf Rückflußtemperatur und rührt zur Vervollständigung der Decarboxylierung noch 4 Stunden unter Rückfluß. Danach zieht man das Lösungsmittel im Vakuum ab. Zurück bleiben 270 g (97% der Theorie) Anil in Form eines farblosen Pulvers mit dem Schmelzpunkt 45—47°C.

Beispiel 3b

Eine Lösung von 101,5 g (0,5 Mol) 4-Fluor-3-brombenzaldehyd und 46,5 g (0,5 Mol) Anilin in 250 ml Toluol wird 1 Stunde am Wasserabscheider gekocht. Dann destilliert man das Lösungsmittel im Vakuum ab. Zurück bleiben 129 g (93% der Theorie) 4-Fluor-3-brom-benzaldehydanil in Form farbloser Kristalle mit dem Schmelzpunkt 47—49°C.

Analog einem der beiden Beispiele 3a bzw. 3b können die folgenden Verbindungen der Formel

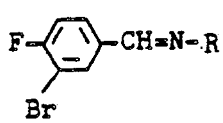

hergestellt werden:

| R | Schmelzpunkt (°C) |
|---|---|
| 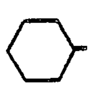 | teilkristallin |
| 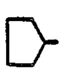 | teilkristallin |
| 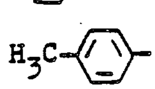 | |
| 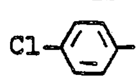 | |

Beispiel 4

4-Fluor-3-brombenzaldehyddimethylacetal ("Eintopf"-Verfahren aus Glyoxylsäure)

Zu einer Mischung aus 1300 ml Toluol oder Chlorbenzol und 247 g 4-Fluor-3-brom-phenylglyoxylsäure tropft man bei 70—80°C 102,3 g (1,1 Mol) frisch destilliertes Anilin. Anschließend erhitzt man die Reaktionsmischung auf Rückflußtemperatur und rührt zur Vervollständigung der Decarboxylierung noch 4 Stunden unter Rückfluß, danach zieht man das Lösungsmittel im Wasserstrahlvakuum ab und destilliert bei 0,5 bis 0,01 Torr an. Der Rückstand wird in 1000 ml wasserfreiem Methanol gelöst und bei 20—25°C 54 g Schwefelsäure-Monohydrat zugetropft. Man rührt 4 Stunden bei Raumtemperatur, dampft das Methanol im Wasserstrahlvakuum ab, nimmt den Rückstand in Toluol auf, saugt ab, und wäscht das Filtrat einmal mit verdünnter Kaliumcarbonat-Lösung. Nach Trockenen der organischen Phase mit Natriumsulfat engt man im Wasserstrahlvakuum ein und destilliert bei 0,1 Torr. Nach einem Vorlauf bei 35—40°C destilliert man das Acetal bei 65—70°C ab. Man erhält auf diese Weist 209 g (84% der Theorie) eines farblosen Öles.

Beispiel 5

4-Fluor-3-brombenzaldehyddimethylacetal aus Anil

Zu einer Lösung von 27,8 g (0.1 Mol) 4-Fluor-3-brombenzaldehydanil in 100 ml absol. Methanol tropft man bei 20—25°C 5,4 g (0,055 Mol) wasserfreie Schwefelsäure und rührt das Gemisch 4 Stunden bei Raumtemperatur nach. Dann wird das Lösungsmittel im Vakuum abgezogen, der Rückstand mit 200 ml Toluol versetzt und vom Ungelösten abgesaugt. Das Filtrat wird mit 50 ml 5%iger Kaliumcarbonatlösung gewaschen, über Natriumsulfat getrocknet und eingedampft. Den Rückstand destilliert man im Vakuum. Man erhält so 22,9 g (92% der Theorie) Acetal in Form eines farblosen Öles mit dem Siedepunkt 79°C/0,5 Torr.

In analoger Weise können die folgenden Verbindungen hergestellt werden:

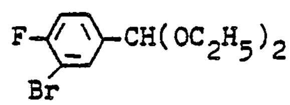

Siedepunkt
7o-72°C/o,2 mm Hg

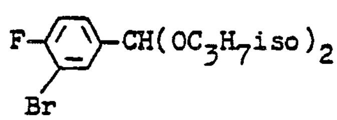

Siedepunkt
67-7o°C/o,1 mm Hg

**Patentansprüche**

1. Verfahren zur Herstellung von 3-Brom-4-fluor-benzaldehyd-acetalen der Formel (I)

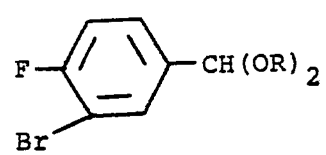

(I)

in welcher die Reste

R einzeln für $C_1$—$C_4$-Alkyl oder zusammen für $C_2$—$C_5$-Alkandiyl stehen, dadurch gekennzeichnet, daß man

(a) 3-Brom-4-fluor-benzaldimine der Formel (II)

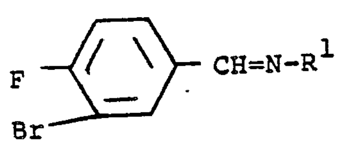

(II)

in welcher

$R^1$ für gegebenenfalls verzweigtes und/oder gegebenenfalls cyclisches und/oder gegebenenfalls substituiertes Alkyl oder für gegebenenfalls substituiertes Aryl steht, mit Alkoholen bzw. Alkandiolen der Formel (III)

$$(HO)_n—R \qquad (III)$$

in welcher

R die oben angegebene Bedeutung hat und

n für 1 oder 2 steht, in Gegenwart von Schwefelsäure bei Temperaturen zwischen 0 und 100°C umsetzt, oder

(b) 3-Brom-4-fluor-phenylglyoxylsäure der Formel (IV)

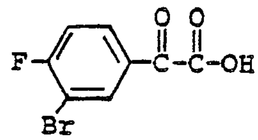

(IV)

mit Aminen der Formel (V)

$$H_2N—R^1 \quad (V)$$

in welcher

$R^1$ die oben angegebene Bedeutung hat, gegebenenfalls in Gegenwart eines Verdünnungsmittels bei Temperaturen zwischen 0 und 200°C umsetzt und die hierbei erhaltenen Rohprodukte, welche die Verbindungen der Formel (II) enthalten, mit Alkoholen der Formel (III) in Gegenwart katalytischer Mengen Schwefelsäure bei Temperaturen zwischen 0 und 100°C umsetzt.

2. 3-Brom-4-fluor-benzaldimine der Formel (II)

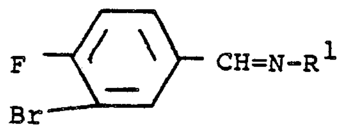

(II)

in welcher

$R^1$ für gegebenenfalls verzweigtes, gegebenenfalls cyclisches und gegebenenfalls substituiertes Alkyl oder für gegebenenfalls substituiertes Aryl steht.

3. Verfahren zur Herstellung der neuen Verbindungen der Formel (II)

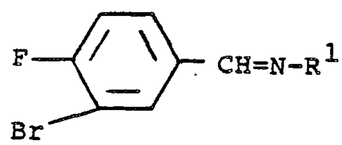

(II)

dadurch gekennzeichnet, daß man

(a) 3-Brom-4-fluor-benzaldehyd der Formel (VI)

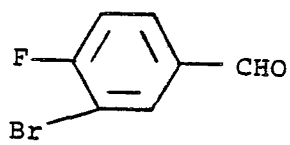

(VI)

mit Aminen der Formel (V) (oben) gegebenenfalls in Gegenwart eines Verdünnungsmittels umsetzt, oder

(b) 3-Brom-4-fluor-phenylglyoxylsäure der Formel (IV) (oben) mit Aminen der Formel (V) (oben) gegebenenfalls in Gegenwart eines Verdünnungsmittels umsetzt.

**Revendications**

1. Procédé de préparation d'acétals du 3-bromo-4-fluoro-benzaldéhyde répondant à la formule I

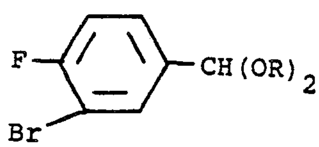

(I)

dans laquelle

les symboles R représentent individuellement des groupes alkyle en $C_1—C_4$ ou, ensemble, un groupe alcane-diyle en $C_2—C_5$ caractérisé en ce que

(a) on fait réagir des 3-bromo-4-fluoro-benzaldimines de formule II

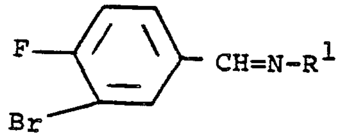

(II)

dans laquelle

$R^1$ représente un groupe alkyle éventuellement ramifié et/ou éventuellement cyclique et/ou éventuellement substitué ou un groupe aryle éventuellement substitué, avec des alcools ou des alcanediols respectivement de formule III

$$(HO)_n—R \qquad (III)$$

dans laquelle

R a les significations indiquées ci-dessus et

n est égal à 1 ou 2, en présence d'acide sulfurique à des températures de 0 à 100°C, ou bien

(b) on fait réagir l'acide 3-bromo-4-fluoro-phénylglyoxylique de formule IV

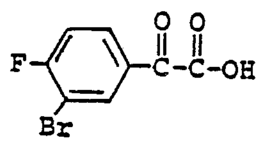

(IV)

avec des amines de formule V

$$H_2N—R^1 \qquad (V)$$

dans laquelle $R^1$ a les significations indiquées ci-dessus, éventuellement en présence d'un diluant, à des températures de 0 à 200°C, et on fait réagir les produits bruts ainsi obtenus, qui contiennent les composés de formule II, avec les alcools de formule III en présence de quantités catalytiques d'acide sulfurique à des températures de 0 à 100°C.

2. 3-bromo-4-fluoro-benzaldimines de formules II

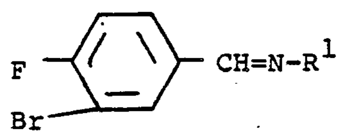

(II)

dans laquelle

$R^1$ représente un groupe alkyle éventuellement ramifié, éventuellement cyclique et éventuellement substitué ou un groupe aryle éventuellement substitué.

3. Procédé de préparation des nouveaux composés de formule II

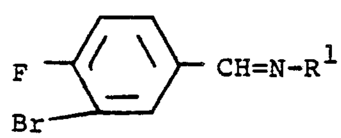

(II)

caractérisé en ce que

(a) on fait réagir le 3-bromo-4-fluoro-benzaldéhyde de formule VI

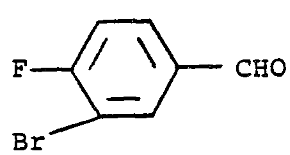

(VI)

avec des amines de formule V ci-dessus, éventuellement en présence d'un diluant, ou bien

(b) on fait réagir l'acide 3-bromo-4-fluoro-phénylglyoxylique de formule IV ci-dessus avec des amines de formule V ci-dessus, éventuellement en présence d'un diluant.

**Claims**

1. Process for the preparation of 3-bromo-4-fluoro-benzaldehyde acetals of the formula (I)

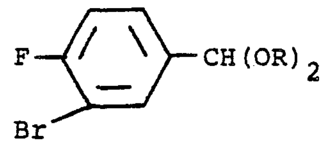

(I)

in which

the radicals R individually represent $C_1$—$C_4$-alkyl or together represent $C_2$—$C_5$-alkanediyl, characterised in that

(a) 3-bromo-4-fluoro-benzaldimines of the formula (II)

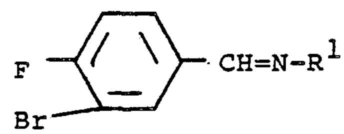

(II)

in which

$R^1$ represents optionally branched and/or optionally cyclic and/or optionally substituted alkyl or optionally substituted aryl, are reacted with alcohols or alkanediols of the formula (III)

$$(HO)_n—R \qquad (III)$$

in which

R has the abovementioned meaning and

n represents 1 or 2, in the presence of sulphuric acid at temperatures between 0 and 100°C, or

(b) 3-bromo-4-fluoro-phenylglyoxylic acid of the formula (IV)

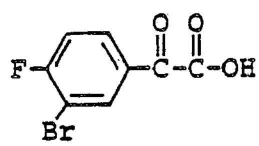

is reacted with amines of the formula (V)

$$H_2N—R^1 \qquad (V)$$

in which

$R^1$ has the abovementioned meaning, if appropriate in the presence of a diluent at temperatures between 0 and 200°C and the crude products thereby obtained, which contain the compounds of the formula (II), are reacted with alcohols of the formula (III) in the presence of catalytic amounts of sulphuric acid at temperatures between 0 and 100°C.

2. 3-Bromo-4-fluoro-benzaldimines of the formula (II)

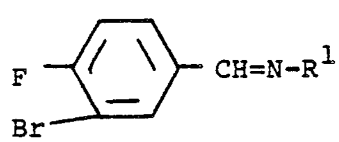

(II)

in which

$R^1$ represents optionally branched, optionally cyclic and optionally substituted aryl or optionally substituted aryl.

3. Process for the preparation of the new compounds of the formula (II)

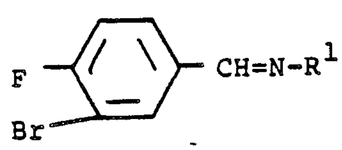

(II)

characterised in that

(a) 3-bromo-4-fluoro-benzaldehyde of the formula (VI)

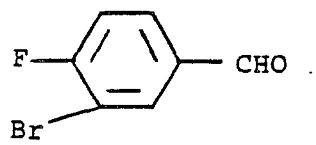

(VI)

is reacted with amines of the formula (V) (above), if appropriate in the presence of a diluent, or

(b) 3-bromo-4-fluoro-phenylglyoxylic acid of the formula (IV) (above) is reacted with amines of the formula (V) (above), if appropriate in the presence of a diluent.